# EUROPEAN PATENT SPECIFICATION

(11) **EP 1 792 921 B1**
(45) Date of publication and mention of the grant of the patent: **12.08.2015**
(21) Application number: 06256024.8
(22) Date of filing: 24.11.2006
(51) Int. Cl.: C08F 20/00, C08F 20/60, C08F 22/00, C08F 22/38, C08F 26/00

(54) **Aqueous polymer dispersons for stabilizing actives**
Wässerige Polymerdispersionen zur Stabilisierung von Aktivstoffen
Dispersions polymères aqueuses pour stabiliser des actifs

(30) Priority: 01.12.2005 US 741323 P
(43) Date of publication of application: 06.06.2007
(73) Proprietor: Rohm and Haas Company, Philadelphia, PA 19106-2399 (US)
(72) Inventor: Lau, Willie, Lower Gwynedd Pennsylvania 19002 (US); Solomon, Robert D., Souderton Pennsylvania 18964 (US); Sheng, Miao-Hsun Li, Lower Gwynedd Pennsylvania 19002 (US)
(74) Representative: Kent, Venetia Katherine

(56) References cited:
- EP-A2- 0 460 385
- WO-A-02/38639
- CA-A- 750 890

## Description

The present invention relates to aqueous polymer dispersions and polymers useful for stabilizing one or more active ingredients. In particular, the invention is an aqueous polymer dispersion that has utility in stabilizing one cr more active ingredients. More particularly, the invention is directed to aqueous polymer dispersions that stabilize water soluble active ingredients, partially water soluble active ingredients and water insoluble active ingredients and combinations thereof.

U. S. Pat. No. 5,142,010 discloses a polymer composition that prevents microbial growth through the presence of strong biocidal groups chemically bonded to functional groups of the polymer. It discloses an antimicrobial composition comprising a polymer, including copolymers, terpolymers, and oligomers having pendent-active functional groups consisting of common amine substituents. One problem with such polymers is that the microbiocide product is intended to remain in the polymer composition to prevent growth of microorganisms on a substrate. It is desirable to provide low molecular weight polymer compositions that stabilize one or more active ingredients that are water soluble, partially water soluble, water insoluble and combinations thereof. Combining the low molecular weight polymer stabilized active ingredient(s) with a chemically and/or structurally related higher molecular weight polymer then effects release of the one or more active ingredients from the polymer mixture.

Accordingly, the invention provides an aqueous dispersion comprising: (a) from 5 to 99.9 % by weight, based on the weight of solids in the aqueous dispersion, of one or more polymers having a number average molecular weight less than 10,000; and (b) from 0.1 to 95 % by weight, based on the weight of solids in the aqueous dispersion, of one or more active ingredients selected from the group consisting of water soluble active ingredients, partially water soluble active ingredients, water insoluble active ingredients and combinations thereof.

In one embodiment the invention provides an aqueous dispersion comprising: (a) from 5 to 49.9 % by weight, based on the weight of solids in the aqueous dispersion, of one or more polymers having a number average molecular weight less than 10,000; (b) from 0.1 to 45 % by weight, based on the weight of solids in the aqueous dispersion, of one or more active ingredients selected from the group consisting of water soluble active ingredients, partially water soluble active ingredients, water insoluble active ingredients and combinations thereof; and (c) from 94.9 to 5.1% by weight, based on the weight of solids in the aqueous dispersion, of one or more film forming polymers having a number average molecular greater than 10,000; wherein the one or more active ingredients are partitioned in (a) prior to combining the components and water.

The invention also provides a method for releasing one or more active ingredients from an aqueous dispersion composition comprising: a) preparing a first aqueous dispersion comprising from 5 to 49.9 % by weight, based on the weight of solids in the aqueous dispersion composition, of one or more polymers having a number average molecular weight less than 10,000; b) combining the first aqueous dispersion with from 0.1 to 45% by weight, based on the weight of solids in the aqueous dispersion composition, of one or more active ingredients selected from the group consisting of water soluble active ingredients, partially water soluble active ingredients, water insoluble active ingredients and combinations thereof; and c) combining the first aqueous dispersion with a second aqueous dispersion from 94.9 to 5.1% by weight, based on the weight of the aqueous dispersion composition, of one or more film forming polymers having a number average molecular greater than 10,000; wherein the one or more active ingredients are partitioned in the first aqueous dispersion prior to combining with the second aqueous dispersion; and d) drying the mixture of aqueous dispersions to form a film or a coating.

As used herein, the term "dispersion" refers to a physical state of matter that includes at least two distinct phases, wherein a first phase is distributed in a second phase, with the second phase being a continuous medium including but not limited to water. An aqueous polymer dispersion is a dispersion containing a first phase distributed in an aqueous second phase that is predominately water. As used herein, the term "stabilizing" refers to controlling or maintaining a polymer particle size or the particle size of one or more active ingredients incorporated in a polymer. The term "stabilizing" also refers to preventing crystallization or recrystallization of one or more active ingredients incorporated in a polymer. Alternatively, the term "stabilizing" as it pertains to one or more active ingredients stabilized by an aqueous polymer dispersion refers to the one or more active ingredients exhibiting the following behavior including but not limited to for example dissolving, encapsulating, partitioning, swelling and combinations thereof in the aqueous polymer dispersion. As used herein, the terms water soluble, partially water soluble and water insoluble are defined as disclosed in U.S. Patent No. 5,521,266. As used herein active ingredients refers to any compound having biological activity, including pharmaceutical activity, including compounds having a chemical/physical response and including synthetic, semi-synthetic and naturally occurring compounds.

Unless otherwise specified, the term particle size as used herein refers to the number average particle diameter as determined using a capillary hydrodynamic fractionation apparatus, such as the Matec CHDF-2000 apparatus (Matec Applied Sciences, MA) with ultraviolet detection at 200 nm. Particle size standards are provided by National Institute of Standards and Technology (NIST) traceable polystyrene standards of 50 to 800 nm, such as supplied by Duke Scientific Corporation, CA. Particle size was also determined by optical microscopy or using a Brookhaven Instruments Corp. 90Plus Particle Size Analyzer. Solids were determined by weight loss after 40 minutes at 150°C. Molecular weight distribution was determined by GPC analysis on a Polymer labs Mixed C 300 x 7.5 mm column connected to an HP1100 auto-sampler and pump equipped with a Polymer Labs evaporative light scattering detector using polystyrene standards. Films prepared from the aqueous dispersions were characterized by electron microscopy, including transmission electron microscopy (TEM) and scanning probe microscopy (SPM, AFM). Whenever appropriate, particle size is measured by optical microscopy (Leitz Orthoplan with 100x oil immersion lens). Domain sizes are determined by Transmission Electron Microscopy with sample obtained from cryogenic microtoning of the film and RuO₄ staining. The domain size can also be inferred from the particle size of the aqueous dispersion of active ingredients in the low molecular polymer matrix.

Unless otherwise specified, the term Mn, as used herein, refers to the number average molecular weight as determined by size exclusion chromatography (SEC) using EasiCal PS-2® polystyrene standards supplied by Polymer Laboratories.

Unless otherwise specified the term Mw, as used herein, refers to the weight average molecular weight as determined by SEC using EasiCal PS-2® polystyrene standards supplied by Polymer Laboratories.
The term Tg as used herein refers to the glass transition temperature of polymers as determined using the Fox equation (T.G. Fox, Bull. Am. Physics Soc., Volume 1, Issue No. 3, page 123(1956)). By "measured Tg," as used herein, is meant the glass transition temperature as determined by differential scanning calorimetry (DSC) using a heating rate of 10° C / minute, taking the mid-point in the heat flow versus temperature transition as the Tg value.

As used herein, the use of the term "(meth)" followed by another term such as acrylate refers to both acrylates and methacrylates. For example, the term "(meth)acrylate" refers to either acrylate or methacrylate; the term "(meth)acrylic" refers to either acrylic or methacrylic; the term "(meth)acrylic acid" refers to either acrylic acid or methacrylic acid; and the term "(meth)acrylamide" refers to either acrylamide or methacrylamide.

High molecular weight aqueous polymer dispersions useful in accordance with the invention comprise polymer particles having a weight average molecular weight polymer greater than 10,000, including up to 100,000, also including up to 500,000 including up to 1,000,000 and including less than 2,000,000. Suitable high molecular weight polymers include but are not limited to emulsion polymers.

Low molecular weight aqueous polymer dispersions (also referred to as "oligomers" or "oligomer dispersions") comprise polymer particles having a number average molecular weight polymer less than 10,000, including less than 5,000 and including less than 3,000. The Mn of oligomer dispersions also is between 1,000 and 10,000. Suitable low molecular weight polymers include but are not limited to emulsion polymers. The molecular weight of oligomer dispersions are controlled by any means known to the art.

According to one embodiment, the chain transfer agent is selected from mercaptans, polymercaptans, thioesters, halogenated compounds, organic solvents including but not limited to alcohols, isopropanol, isobutanol and combinations thereof. In some preferred embodiments the molecular weight of oligomer dispersions is controlled through the use of linear or branched C₄-C₂₂ alkyl mercaptans such as n-dodecyl mercaptan and t-dodecyl mercaptan. It is also contemplated that the molecular weight of oligomer dispersions may be controlled through the use of catalytic chain transfer agents, such as the cobalt compounds described in U.S. Patents Nos. 5,962,609 and 4,746,713. According to a separate embodiment, the molecular weight of the oligomer is controlled by isopropyl alcohol as the solvent a solution polymerization.

Low molecular weight polymer dispersions or oligomer dispersions are formed by any means known in the art including, for example, bulk, solution, emulsion, mini-emulsion, micro-emulsion, or suspension polymerization processes. Oligomers are also be formed by bulk or solution polymerization. For solution polymerization, the chain transfer agent may be the solvent such as isopropyl alcohol. The low molecular weight polymer is isolated by evaporation of the sol.vent. The low molecular weight polymer dispersions or "oligomer" dispersions may be prepared by emulsification of the low molecular weight polymer in water. According to one embodiment, the polymer having a Mn <10,000 or "oligomer" is formed by the free radical initiated polymerization of one or more ethylenically unsaturated monomers. According to a separate embodiment, other forms of initiation, including anionic initiation, are contemplated. In one such embodiment, the oligomer is 1'ormed by high temperature oligomerization processes as disclosed in U.S. Patent No. 5,710,227. If oligomers are formed by means other than aqueous dispersion processes, they are converted to an aqueous dispersion by techniques known to the art. According to a separate embodiment, the oligomer dispersion is prepared as an aqueous dispersion and water is removed, creating an oligomer oil. When acrylic monomers are used to prepare the low molecular weight polymer and acrylic oil (AO) is prepared. According to a separate embodiment, the oligomer dispersion is prepared by solution polymerization which directly produces an oligomer oil. When acrylic monomers are used to prepare the low molecular weight polymer and acrylic oil (AD) is prepared. The oligomer can be re-emulsified or converted to an aqueous dispersion by conventional techniques known in the art. According to a separate embodiment, the oligomer is prepared as polymer solids and emulsified to form an aqueous dispersion by conventional techniques known in the art.

Advantages of preparing the oligomer as an oil are that particle size is controlled and when combined with one or more active ingredients that are partially soluble or water insoluble the domain size of the active ingredients is also controlled. The distribution and location of the dispersion in a polymer matrix such as a film is also controlled.

Another advantage of the invention is that low molecular weight polymers provide a method for controlling and adjusting the loading of one or more active ingredients in the aqueous dispersion. High amounts of one or more actives (up to 95 % by weight) to low amounts (< 0.5 % by weight) of one or more highly active ingredients can be loaded into the polymers or prepared as a polymer composition.

Another advantage of the invention is that low molecular weight polymers provide a method for controlling the domain size of the active ingredient from 1000 microns to 1 nm, including AI domains below 10 microns in size and including AI domains below 1 micron in size.

Another advantage of the invention is that low molecular weight polymers provides a method for controlling morphology of a polymer film or composite film with regard to the distribution and location of the active ingredient such that the AI domains are homogeneously distributed in the polymer film matrix, or are concentrated at the surface of the film, or distributed as a gradient in the film.

Another advantage of the invention is that low molecular weight polymers provides a method for including a plurality of domains of different active ingredients using one or more polymers having a low molecular weight.

Another advantage of the invention is that low molecular weight polymers provides a method of controlling the releasing rate and profile of the active ingredients using polymers of the invention through the control of particle size, domain size, domain distribution, and concentration.

The high molecular weight and low molecular weight polymers of the invention include polymers formed by the polymerization of one or more ethylenically unsaturated monomers, condensation polymers, hybrid polymers containing both condensation polymer and addition polymer. Condensation polymers are polymers that are not formed by the addition polymerization of ethylenically unsaturated monomers, and include, for example, polyurethanes, polyureas, polyesters, polyamides, alkyds, polycarbonates, polysilicones such as the condensation product of hexamethylcyclotrisiloxane (D₃); octamethylcyclotetrasiloxane (D₄), and decamethylcyclopentasiloxane (D₅); polyalkyl oxides such as polyethylene oxide; polyimides; polysulfones; polyacetals; and biopolymers such as polyhydroxy alkanoates, polypeptides, and polysaccharides.

Both high molecular weight and low molecular weight polymers useful in accordance with the invention are prepared by the polymerization of one or more ethylenically unsaturated monomers and are polymerized by any means known in the art including solution, emulsion, mini-emulsion, microemulsion, or suspension polymerization processes. Preferred is emulsion or mini-emulsion. The practice of emulsion polymerization is discussed in detail in D.C. Blackley, Emulsion Polymerization (Wiley, 1975) and H. Warson, The Applications of Synthetic Resin Emulsions, Chapter 2 (Ernest Benn Ltd., London 1972).

In those embodiments of the invention utilizing emulsion or mini-emulsion polymerization processes conventional surfactants may be used such as, for example, anionic and/or nonionic emulsifiers such as, for example, alkali metal or ammonium salts of alkyl, aryl, or alkylaryl sulfates, sulfonates or phosphates; alkyl sulfonic acids; sulfosuccinate salts; fatty acids; ethylenically unsaturated surfactant monomers; and ethoxylated alcohols or phenols. The amount of surfactant used is usually 0.1% to 6% by weight, based on the weight of monomer. Either thermal or redox initiation processes may be used. The reaction temperature is typically maintained at a temperature lower than 100 °C throughout the course of the reaction. Preferred is a reaction temperature between 30 °C and 95 °C, more preferably between 50 °C and 90 °C. The monomer mixture may be added neat or as an emulsion in water. The monomer mixture may be added in one or more additions or continuously, linearly or not, over the reaction period or combinations thereof.

According to one embodiment, both high and low molecular weight polymers of the invention are formed by polymerization of ethylenically unsaturated monomers using conventional free radical initiators such as, for example, ammonium or sodium persulfate, hydrogen peroxide, sodium peroxide, potassium peroxide, t-butyl hydroperoxide, cumene hydroperoxide, ammonium and/or alkali metal persulfates, sodium perborate, perphosphoric acid and salts thereof, potassium permanganate, and ammonium or alkali metal salts of peroxydisulfuric acid, typically at a level of 0.01% to 3.0% by weight, based on the weight of total monomer. Redox systems using the same initiators (alternatively referred to as "oxidants" herein) coupled with a suitable reductant such as, for example, sodium sulfoxylate formaldehyde, ascorbic acid, isoascorbic acid, alkali metal and ammonium salts of sulfur-containing acids, such as sodium sulfite, bisulfite, thiosulfate, hydrosulfite, sulfide, hydrosulfide or dithionite, formadinesulfinic acid, hydroxymethanesulfonic acid, sodium 2-hydroxy-2-sulfinatoacetic acid, acetone bisulfite, amines such as ethanolamine, glycolic acid, glyoxylic acid hydrate, lactic acid, glyceric acid, malic acid, tartaric acid and salts of the preceding acids may be used. Redox reaction catalyzing metal salts of iron, copper, manganese, silver, platinum, vanadium, nickel, chromium, palladium, or cobalt may be used.

Ethylenically unsaturated nonionic monomers useful in preparing polymers having Mn > 10,000 and polymers having Mn <10,000 in accordance with the invention include, for example, (meth)acrylic ester monomers including methyl acrylate, ethyl acrylate, butyl acrylate, 2-ethylhexyl acrylate, decyl acrylate, lauryl acrylate, methyl methacrylate, butyl methacrylate, isodecyl methacrylate, lauryl methacrylate, hydroxyethyl methacrylate, hydroxypropyl methacrylate, styrene, substituted styrenes, ethylene, butadiene; vinyl acetate, vinyl butyrate and other vinyl esters; vinyl monomers such as vinyl chloride, vinyl toluene, and vinyl benzophenone; and vinylidene chloride.

Ethylenically unsaturated acid monomers useful in preparing polymers having Mn > 10,000 and polymers having Mn <10,000 in accordance with the invention include, for example, acrylic acid, methacrylic acid, crotonic acid, itaconic acid, fumaric acid, maleic acid, monomethyl itaconate, monomethyl fumarate, monobutyl fumarate, maleic anhydride, 2-acrylamido-2-methylpropane sulfonic acid, vinyl sulfonic acid, styrene sulfonic acid, 1-allyloxy-2-hydroxypropane sulfonic acid, alkyl allyl sulfosuccinic acid, sulfoethyl (meth)acrylate, phosphoalkyl (meth)acrylates such as phosphoethyl (meth)acrylate, phosphopropyl (meth)acrylate, and phosphobutyl (meth)acrylate, phosphoalkyl crotonates, phosphoalkyl maleates, phosphoalkyl fumarates, phosphodialkyl (meth)acrylates, phosphodialkyl crotonates, and allyl phosphate.

In some embodiments of the invention polymers formed by the polymerization of ethylenically unsaturated monomers may contain copolymerized multi-ethylenically unsaturated monomers such as, for example, allyl methacrylate, diallyl phthalate, 1,4-butylene glycol dimethacrylate, 1,2-ethylene glycol dimethacrylate, 1,6-hexanediol diacrylate, and divinyl benzene.

In some embodiments of the invention it is desirable to incorporate into one or more of the polymeric components functional monomers which impart specialized performance to the aqueous dispersion. An example would be the inclusion of monomers bearing functional groups which impart improved adhesion to certain substrates. Ethylenically unsaturated monomers bearing such functional groups include but are not limited to for example, vinyl acetoacetate, acetoacetoxyethyl (meth)acrylate, acetoacetoxypropyl (meth)acrylate, allyl acetoacetate, acetoacetoxybutyl (meth)acrylate, 2,3-di(acetoacetoxy)propyl (meth)acrylate, vinyl acetoacetamide, and acetoacetoxyethyl (meth)acrylamide

According to one embodiment, when emulsion polymerization of one or more monomers is employed, chain transfer agents such as, for example, halogen compounds such as tetrabromomethane; allyl compounds; or mercaptans such as alkyl thioglycolates, alkyl mercaptoalkanoates, and C₄-C₂₂ linear or branched alkyl mercaptans may be used to lower the molecular weight of the polymers formed by the polymerization of ethylenically unsaturated monomers and/or to provide a different molecular weight distribution than would otherwise have been obtained with any free-radical-generating initiator(s). According to a separate embodiment when solution polymerization of one or more monomers is employed, organic solvents including but not limited alcohols such as isopropanol are used as chain transfer agents to regulate molecular weight.

According to one embodiment, the oligomers having utility in accordance with the invention have partial or no solubility in water. By this we mean that the dissolved concentration of oligomer dispersions in water is no greater than 5 weight % at 25-50°C at any pH between 2 and 12; preferably no greater than 2 weight %; and more preferably no greater than 0.1 weight %.

The aqueous dispersion, based on polymer solids, comprises: a) from 5 to 99.9 % by weight, based on the weight of solids in the aqueous dispersion, of one or more polymers having a number average molecular weight less than 10,000: b) from 0.1 to 95% by weight, based on the weight of solids in the aqueous dispersion, of one or more active ingredients selected from the group consisting of water soluble active ingredients, partially water soluble active ingredients, water insoluble active ingredients and combinations thereof; and c) from 30% to 95% by weight of water.

Alternatively, a composition based on weight percent, is prepared in accordance with the invention that comprises: (a) from 5 to 99.9 % by weight, based on the weight of the composition, of one or more polymers having a number average molecular weight less than 10,000; (b) from 0.1 to 95 % by weight, based on the weight of the composition, of one or more active ingredients selected from the group consisting of water soluble active ingredients, partially water soluble active ingredients, water insoluble active ingredients and combinations thereof.

In some embodiments the inventive dispersions may be formed by blending an aqueous dispersion comprising polymer dispersions and oligomer dispersions with an aqueous dispersion comprising other polymer dispersions. By blending is meant any means of combining or mixing at least an aqueous dispersion comprising polymer dispersions and oligomer dispersions with an aqueous dispersion comprising other polymer dispersions. Means of blending may include adding a dispersion comprising a polymer dispersion and an oligomer dispersion to an aqueous dispersion comprising another polymer dispersion or by adding a dispersion comprising the latter polymer dispersion to a dispersion comprising the former polymer dispersion and the oligomer dispersion. The dispersions may be combined in batch, semicontinuous, or continuous fashion.

According to one embodiment, aqueous low molecular weight polymer dispersions and oligomer dispersions are prepared by free radical aqueous polymerization in the presence of a macromolecular organic compound having a hydrophobic cavity, as disclosed in U.S. Patent No. 5,521,266. The macromolecular organic compound having a hydrophobic cavity useful in the method of the invention include cyclodextrin and cyclodextrin derivatives; cyclic oligosaccharides having a hydrophobic cavity such as cycloinulohexose, cycloinuloheptose, and cycloinuloctose; calyxarenes; and cavitands.

The cyclodextrin and cyclodextrin derivatives useful in the method of the invention are limited only by the solubility of the cyclodextrin and cyclodextrin derivative selected under the particular polymerization conditions. Suitable cyclodextrins useful in the method of the present invention include, but are not limited to, a-cyclodextrin, b-cyclodextrin and g-cyclodextrin. Suitable cyclodextrin derivatives useful in the method of the present invention include, but are not limited to, the methyl, triacetyl hydroxypropyl and hydroxyethyl derivatives of a-cyclodextrin, b-cyclodextrin and g-cyclodextrin. The preferred cylodextrin derivative is methyl-b-cyclodextrin.

The cyclic oligosaccharides having a hydrophobic cavity, such as cycloinulohexose, cycloinuloheptose, useful in the method of the invention are described by Takai et al., Journal of Organic Chemistry, 1994, volume 59, number 11, pages 2967-2975.

The calixarenes useful in the method of the invention are described in U.S. Patent No. 4,699,966, International Patent Publication WO 89/08092 and Japanese patent publications 1988/197544 and 1989/007837.

The cavitands useful in the method of the invention are described in Italian patent application no. 22522 A/89 and Moran et al., Journal of the American Chemical Society, volume 184, 1982, pages 5826-5828.

The use of a macromolecular organic compound having a hydrophobic cavity will be particularly useful when any of polymer and oligomer dispersions are formed by aqueous free radical polymerization and when one or more of the monomers and/or chain transfer agents used in the polymerization has a water solubility at 25-50°C of no greater than 200 millimoles/liter; no greater than 50 millimoles/liter.

When any of the polymer and oligomer dispersions are formed by aqueous free radical polymerization and when one or more of the monomers and/ or chain transfer agents used in the polymerization has a water solubility at 25-50°C of no greater than 200 millimoles/liter; no greater than 50 millimoles/liter it may also be useful to introduce the monomers and/ or chain transfer agents to the polymerization in the form of a monomer emulsion with an average droplet size of less than 50; less than 25 microns.

The aqueous oligomer dispersion is combined with of one or more active ingredients selected from the group consisting of water soluble active ingredients, partially water soluble active ingredients, water insoluble active ingredients and combinations thereof. According to some embodiments, active ingredients (AIs) are any compounds having biological activity. According to other embodiments, active ingredients are compounds having pharmaceutical activity. According to other embodiments, AIs are biologically active or pharmaceutically active compounds that are applied to mammalian skin for a topical effect or to be absorbed through the skin for a systemic effect. According to other embodiments, AIs are compounds applied to a loci or an environment of use. According to other embodiments, AIs are compounds having a physicochemical response when applied to a loci or an environment of use. According to other embodiments, AIs are compounds that protect surfaces, including but not limited to for example, mammalian skin.

Active ingredients used in accordance to the present invention are water soluble, partially water soluble, water insoluble and combinations thereof. Water soluble, partially water soluble, water insoluble and mixtures thereof in the form of AIs that are liquids or solids and that are amorphous, crystalline, semi-crystalline and combinations thereof.

According to one embodiment, the aqueous oligomer dispersion is combined with one or more water soluble AIs, wherein the AIs are amorphous, crystalline, semi-crystalline and combinations thereof. The water soluble AIs are combined with the aqueous oligomer dispersion in any suitable manner, including but not limited to for example, the one or more AIs are present when one or more monomers are polymerized to form the oligomer dispersion, the one or AIs are added to an oligomer dispersion, the oligomer dispersion as solids is dispersed in an aqueous solution including one or more water soluble AIs. The water soluble AIs are either selectively partitioned within the oligomer dispersion or are distributed in the oligomer (oil phase) and the aqueous phase.

According to separate embodiments, the aqueous oligomer dispersion is combined with one or more partially water soluble or water insoluble AIs, wherein the AIs are amorphous, crystalline, semi-crystalline and combinations thereof. The AIs are combined with the aqueous oligomer dispersion in any suitable manner, including but not limited to for example, the one or more AIs are present when one or more monomers are polymerized to form the oligomer dispersion, the one cr more AIs are added to an oligomer dispersion, the oligomer dispersion, including one or more partially water soluble or water insoluble AIs partitioned therein, is dispersed in an aqueous solution. The water soluble AIs maybe selectively partitioned within the oligomer dispersion or may be distributed in the oligomer (oil phase) and the aqueous phase. The AIs are either selectively partitioned within the oligomer dispersion or are distributed in the oligomer (oil phase) and the aqueous phase.

According to one embodiment, the one or more active ingredients are any biologically active agents, including agents having pharmacological activity and immunological activity. The biologically active agents exhibit morphologies selected from crystalline, semi-crystalline, amorphous and combinations thereof. Suitable biologically active agents include but are not limited to for example, antiinflammatory agents; anti-microbial agents; biocides; anti-bacterial agents, antiviral agents including but not limited to Acylovir^{™} (water sol 37°C, 2.5 mg/mL), Lamivudine^{™} (water sol. 20°C, 70 mg/mL), Zidovudine^{™} (water sol. 25° C, 20.1 mg/mL), Zanamivir^{™} (water sol. 20° C, 18 mg/mL), Oseltamivir^{™} (Tamiflu^{™}), Ribavirin^{™}, Pleconaril^{™} and Abacavir^{™} sulfate (water sol. 25° C, 77 mg/mL), Amprenavir^{™} (water sol. 25° C is 0.04 mg/mL), fosamprenavir^{™} (water sol. 25° C, 0.31 mg/mL), and valacyclovir^{™} (water sol. 25°C, 174 mg/mL), anti-retroviral agents, amino acids, peptides, proteins, DNA, RNA, lipids, lyposomes, adrenergic agents; adrenocortical steroids, adrenocortical suppressants, aldosterone antagonists, anabolic agents; analeptic agents; analgesic agents; anesthetic agents; anorectic agents; anti-acne agents; anti-adrenergic agents; anti-allergic agents; anti-amebic agents; anti-anemics; anti-angina agents; anti-arthritic agents; anti-a.sthmatics; anti-atherosclerotic agents; anticholinergics; anticoagulants; anticonvulsants; antidepressants; anti-diabetic agents; anti-diarrheals; anti-diuretics; anti-emetics; antiepileptics; anti-fibrinolytics; anti-fungal agents; anti-hemorrhagics; anti-histamines; anti-hyperlipidemia agents; antihypertensives; anti-hypotensives; anti-infective agents; anti-migraines; anti-mitotics; anti-mycotics, anti-nauseants, anti-neoplastics, anti-neutropenics, anti-parasitics; antiproliferatives; antipsychotics; antirheumatics; antiseborrheics; antisecretories; antispasmodics; antithrombotisc; anti-ulcerative agents; appetite suppressants; blood glucose regulators; bone resorption inhibitors; bronchodilators, cardiovascular agents; cholinergics; depressants; diagnostic aids; diuretics; dopaminergic agents, estrogen receptor agonists; fibrinolytics; fluorescent agents; free oxygen radical scavengers; gastrointestinal motility effecters; glucocorticoids; hair growth stimulants; hemostatics; histamine H2 receptor antagonists; hormones; hypocholesterolemics; hypoglycemics; hypolipidemics; hypotensives; imaging agents; immunizing agents; immunomodulators; immunoregulators; immunostimulants; immunosuppressants; keratolytics; LEIGH agonists; mood regulators; mucolytics; mydriatics; nasal decongestants; neuromuscular blocking agents; neuroprotectives; NMDA antagonists; non-hormonal sterol derivatives; plasminogen activators; platelet activating factor antagonists; platelet aggregation inhibitors; psychotropics; radioactive agents; scabicides; sclerosing agents; sedatives; sedative-hypnotics; selective adenosine Al antagonists; serotonin antagonists; serotonin inhibitors; serotonin receptor antagonists; steroids; thyroid hormones; thyroid inhibitors; thyromimetics; tranquilizers; amyotrophic lateral sclerosis agents; cerebral ischemia agents; Paget's disease agents; unstable angina agents; vasoconstrictors; vasodilators; wound healing agents; xanthine oxidase inhibitors; anti-cancer agents and combinations of biologically active agents.

Suitable biologically active agents also include immunological agents such as allergens, pollens, and antigens from pathogens such as viruses, bacteria, fungi and parasites. The antigens may be in the form of whole inactivated organisms, peptides, proteins, glycoproteins, carbohydrates or combinations thereof. Specific examples of pharmacological or immunological agents that fall within the above-mentioned categories and that have been approved for human use are known by those having skill in the art.

According to other embodiments, the one or more active ingredients of the invention include but are not limited to any compound that exhibits a chemical and/or physical effect. Suitable active ingredients of the invention include, but are not limited to for example, gases, pesticides, herbicides, fragrances, anti-foulants, dyes, salts, oils, inks, cosmetics, catalysts, detergents, UV absorbers, organic compounds, and polymers, flavors, foods, photographic agents, biocides, pharmaceuticals, medicaments, and combinations thereof

The aqueous oligomer dispersion, which includes one or more AIs is combined with a second aqueous polymer dispersion. The resulting aqueous dispersion is a homogeneous dispersion or one that includes domains of oligomer with AIs partitioned therein. According to one embodiment, the resulting aqueous dispersion comprises: (a) from 5 to 49.9 % by weight, based on the weight of solids in the aqueous dispersion, of one or more polymers having a number average molecular weight less than 10,000; (b) from 0.1 to 45 % by weight, based on the weight of solids in the aqueous dispersion, of one or more active ingredients selected from the group consisting of water soluble active ingredients, partially water soluble active ingredients, water insoluble active ingredients and combinations thereof; and (c) from 94.9 to 5.1% by weight, based on the weight of solids in the aqueous dispersion, of one or more film forming polymers having a number average molecular greater than 10,000; wherein the one or more active ingredients (b) are partitioned in (a) prior to combining with (c) and water. When the aqueous dispersion dries and coalesces into a useful article including but not limited to for example, a film, a coating, an adhesive, a pressure sensitive adhesive (PSA), a sealant, a cosmetic, an ointment, a cream or a lotion, the one or more AI become incompatible within the article and diffuse out of the polymer to a polymer substrate interface which includes but is not limited any conventionally known substrate and mammalian skin.

The monomers in both the oligomer dispersion and the polymer dispersion are adjusted according to the water solubility or the water insolubility of the AIs. Water soluble AIs are partitioned into oligomers including acid containing monomers. Partially soluble or water insoluble AIs are partitioned into oligomers including nonionic monomers.

The oligomer dispersion and the polymer dispersion are used to prepare a delivery system. According to one embodiment the system comprises a latex polymer blend. According to one embodiment, one component of the blend is a high molecular weight polymer having good film forming characteristics. The latex composition can be prepared into a suitable article such as a coatir.g or PSA. A second component comprises an oligomer latex which includes one or more AIs partitioned therein. The oligomer latex can also function as a reservoir of active ingredients including water soluble and water insoluble pharmaceutical compositions. When the polymer latex dries into a film, the one or more AI s are released or delivered into a loci or environment of use or mammalian skin.

The aqueous dispersion of one more AIs is also dried by conventional techniques known in the art to provide a dry composition in the form of articles including but limited to form example, films, adhesives, PSAs, sealants, elastomers, and foams. According to some embodiments, compositions of the invention are prepared by drying the aqueous polymer dispersions, including, for example, spray drying, interfacial polymerization, hot melt encapsulation, phase separation encapsulation, freeze drying, solvent evaporation, microencapsulation, solvent removal microencapsulation, coacervation, and low temperature microsphere formation and phase inversion.

The method of releasing the one or more AIs to an environment of use, loci or mammalian skin has utility as a delivery system in the form of articles not limited to for example a patch, an adhesive, a coating, a sealant, a foam, an oil, and medical articles including but not limited to for example, gauzes, devices, sutures, bandages, and adhesives.

Exemplary embodiments of the invention are summarized in the following Examples. For examples 1-5, particle size was determined by optical microscopy or using a Brookhaven Instruments Corp. 90Plus Particle Size Analyzer. Solids were determined by weight loss after 40 minutes at 150°C. Molecular weight distribution was determined by GPC analysis with a Polymer Labs Mixed C 300 x 7.5 mm column connected to an HP1100 auto-sampler and pump equipped with a Polymer Labs evaporative light scattering detector using polystyrene standards. Films prepared from the aqueous dispersions were characterized by electron microscopy, including transmission electron microscopy (TEM) and scanning probe microscopy (SPM, AFM).

**Abbreviations used in the Examples**

| Surfactant | Ethoxylated C6 to C18 alkyl ether sulfate having from 1 to 40 ethylene oxide groups per molecule (30% active in water) |
|---|---|
| LA | Lauryl acrylate |
| BA | Butyl acrylate |
| MAA | Methacrylic acid |
| nDDM | n-Dodecylmercaptan |
| IPA | Isopropyl alcohol |
| Oligomer | Low molecular weight acrylic polymer having Mn < 10,000. |
| Me-β-CD | Methyl-β-Cyclodextrin (BETA W7 M1.8), Wacker Chemicals (USA), Inc., (50.8% active in water) |

### Example 1: Low Molecular Weight Polymer Dispersions by Emulsion Polymerization

Emulsion polymerizations were carried out in a 5-liter round bottom flask with four necks equipped with a mechanical stirrer, temperature control device, condenser, monomer and initiator feed lines and a nitrogen inlet.

Deionized water (1100 g), Methyl-β-cyclodextrin (59.2 g) and surfactant (18.8) were introduced into the reaction flask at room to form a reaction mixture. The contents were heated to 85°C while stirring under nitrogen sweep. A monomer emulsion of deionized water (625 g), surfactant (14.1 g), monomers BA (1050 g, LA (450 g), nDDM (300 g) was prepared separately.

At 85°C, 5.0 grams of ammonium persulfate in 25 grams of water were added to the reaction mixture. An acrylic dispersion (137.6 g at 45% solids) was added as a seed polymer. After the temperature stabilized at 85°C, the monomer emulsion was fed into the reaction mixture over 100 minutes together with an ammonium persulfate solution (1.0 gram in 100 grams of water). At the end of the monomer emulsion feed, the reaction mixture was held at 85°C for 20 minutes, cooled to 75°C, chased with t-butyl hydroperoxide and isoascorbic acid (0.51 g in 10 g of water and 0.27 g in 25 g of water, respectively) in the presence of Fe₂SO₄ (4 g of 0.15% solution). An addition chase was carried out at 60°C.

The acrylic dispersion (example 1A) has a total solids content of 54.1% and a particle size of 304 nm. The weight average molecular weight (Mw) and number average molecular weight (Mn) were measured at 2449 and 1842, respectively.

A second dispersion (example 1B) was prepared similar with BA (900 g), styrene (585 g), MAA (15 g) and nDDM (202.5 g). The particle size of example 1B was measured at 487 nm, solids at 38.3% and Mn at 1500.

### Example 2 Low Molecular Weight Polymer from Emulsion Polymerization

A low molecular weight polymer was isolated from the dispersion of Example 1 upon evaporation of the water as a viscous oil. The oil was filtered through zeolite to obtain a clear oil, referred to as an acrylic oil (AO).

### Example 3 Low Molecular Weight Polymers from Solution Polymerization

Solution polymerizations were carried out in a 3-liter round bottom flask with four necks equipped with a mechanical stirrer, temperature control device, condenser, monomer and initiator feed lines and a nitrogen inlet.

Isopropanol (600 g) was introduced into the reaction flask at room temperature. The content was heated to 82°C under nitrogen sweep. A monomer mixture was prepared containing BA (1800 g) and isopropanol (180 g) was prepared separately. The monomer mixture was fed into the reaction flask over 120 minutes together with an initiator solution containing Trigonox^{™} 125-C75 (72 g) in isopropanol (300 g). The reaction mixture was held at 85°C for 30 minutes at the end of the feed and an initiator solution containing Trigonox^{™} 125-C75 (18 g) in isopropanol (168 g) was added. After 30 minutes at 85°C, another initiator solution containing Trigonox^{™} 125-C75 (18 g) in isopropanol (168 g) was added. The reaction mixture was held at 85°C for 90 minutes and then cooled to room temperature.

The low molecular polymer was isolated by the stripping of the isopropanol using a rotary evaporator to yield clear viscous oil. The Mw and Mn of the polymer were measured by GPC at 5556 and 4190, respectively.

### Example 4 Low Molecular Weight Polymer Dispersion by Solution Polymerization

A stable aqueous dispersion was prepared that included 4g of low molecular weight polymer (Example 3, 100 BA/1.9 iPrOH, Mn 4191), 0.4g of Neodol 45-7, 0.5g of RM8W (17.5% solid) and 5.1g of water. The mixture is sonicated for 1 minute to yield dispersion with a particle size of 0.5-1µ and total solids of ~40%.

### Example 5 Aqueous dispersions of low molecular weight polymer and partially water soluble active ingredients

Aqueous dispersion of Example 1A (10 g) was mixed with methyl salicylate (19 g) under ambient conditions. The aqueous dispersion was viewed under an optical microscope. The particles visibly swelled to from 0.8-1 microns within 30 minutes. The theoretical particle size with quantitative swelling is 830 nm.

Alternatively, a dispersion derived from solution polymerization (example 4) is to prepare the dispersion in a similar manner.

### Example 6 Aqueous dispersion of low molecular weight polymer and water insoluble active ingredient

Ibuprofen^{™} (2.5 g) was dissolved in low acrylic oil (5 g, Example 1A) at 40°C. The oil mixture solution was added to an aqueous solution consisting of water (6 g), Tergital^{™} 15-S-5 (0.5 g) and sodium lauryl sulfate SLS (1 g) at 60°C. The aqueous mixture was sonicated for 1 minute (40% power setting of a maximum 500 W Sonicator Processor, XL) at 60°C to yield an aqueous dispersion of acrylic oil/Ibuprofen in water with particle sizes ~200 nm. The aqueous dispersion as prepared included 16.7% by weight Ibuprofen^{™} and 33.3% acrylic oil by weight in water. The dispersion was stable at room temperature for 30 minuses after which crystal formation in the form of needles can be seen under an optical microscope.

Alternatively, a low molecular weight polymer derived from solution polymerization (Example 3) is combined with Ibuprofen^{™} in a similar manner to form an aqueous dispersion.

### Example 7 Aqueous dispersion of low molecular weight polymer, water insoluble active ingredient and film forming polymers

Aqueous dispersion from Example 4 (6 g) was mixed with a film forming adhesive dispersion, Robond^{™} PS-90 (14 g) to form an aqueous dispersion consist of 5.4% Ibuprofen^{™}, 10.8% acrylic oil and 34.7% of high molecular weight polymer by weight in water. The aqueous dispersion was stable for over a week without crystal formation as viewed under an optical microscope.

### Example 8 A composition of active ingredient in polymer

An aliquot (8 g) of the aqueous dispersion in example 5 was added to an 8 inches diameter Teflon^{™} petri dish and dried under ambient conditions. The final dry composition consists of 70% high molecular weight polymer, 10% Ibuprofen^{™} and 20% acrylic oil. Ibuprofen^{™} was observed in the polymer phase as domains (-125 nm) as observed by Transmission Electron Microscope with sample microtoned under cryogenic conditions and stained with RuO₄.

## Claims

1. An aqueous dispersion comprising: a) from 5 to 99.9% by weight, based on the weight of solids in the aqueous dispersion, of one or more polymers, prepared by the polymerization of one or more ethylenically unsaturated monomers, having a number average molecular weight less than 10,000; and b) from 0.1 to 95% by weight, based on the weight of solids in the aqueous dispersion, of one or more active ingredients that are compounds having biological activity selected from the group consisting of water soluble active ingredients, partially water soluble active ingredients, water insoluble active ingredients and combinations thereof.

2. The aqueous dispersion of Claim 1 wherein : (a) from 5 to 49.9% by weight, based on the weight of solids in the aqueous dispersion, is of said one or more polymers; (b) from 0.1 to 45% by weight, based on the weight of solids in the aqueous dispersion, is of said one or more active ingredients; and further comprising (c) from 94.9 to 5.1 % by weight, based on the weight of solids in the aqueous dispersion, of one or more film forming polymers, prepared by the polymerization of one or more ethylenically unsaturated monomers, having a number average molecular weight greater than 10,000; and wherein the one or more active ingredients are partitioned in (a) prior to combining the components and water.

3. The aqueous dispersion of claim 1 or claim 2 wherein said one or more polymers, prepared by the polymerization of one or more ethylenically unsaturated monomers, having a number average molecular weight less than 10,000 has a number average molecular weight between 1,000 and 10,000.

4. The aqueous dispersion of any of claims 1 to 3 wherein said one or more polymers, prepared by the polymerization of one or more ethylenically unsaturated monomers, having a number average molecular weight less than 10,000 has partial or no solubility in water such that the dissolved concentration of polymer in water in the range 25 to 50°C, and at any pH from pH 2 to pH 12 is no greater than 5 weight %.

5. Use of the aqueous dispersion of claim 2 in the preparation of a film or an adhesive.

6. A method for releasing one or more active ingredients from an aqueous dispersion composition comprising: a) preparing a first aqueous dispersion comprising from 5 to 49.9% by weight, based on the weight of solids in the aqueous dispersion composition, of one or more polymers , prepared by the polymerization of one or more ethylenically unsaturated monomers, having a number average molecular weight less than 10,000; b) combining the first aqueous dispersion with 0.1 to 45% by weight, based on the weight of solids in the aqueous dispersion composition, of one or more active ingredients that are compounds having biological activity selected from the group consisting of water soluble active ingredients, partially water soluble active ingredients, water insoluble active ingredients and combinations thereof; and c) combining the first aqueous dispersion with a second aqueous dispersion of 94.9 to 5.1 % by weight, based on the weight of the aqueous dispersion composition, of one or more film forming polymers, prepared by the polymerization of one or more ethylenically unsaturated monomers, having a number average molecular weight greater than 10,000; wherein the one or more active ingredients are partitioned in the first aqueous dispersion prior to combining with the second aqueous dispersion; and d) drying the mixture of aqueous dispersions to form a film or a coating.

7. The method according to claim 6 wherein a film is formed after drying the combination of the aqueous dispersions.

## Patentansprüche

1. Eine wässrige Dispersion, beinhaltend: a) zu von 5 bis 99,9 Gew.-%, bezogen auf das Gewicht der Feststoffe in der wässrigen Dispersion, ein oder mehrere durch die Polymerisation eines oder mehrerer ethylenisch ungesättigter Monomere hergestellte Polymere mit einem Zahlenmittel der Molmasse von weniger als 10 000; und b) zu von 0,1 bis 95 Gew.-%, bezogen auf das Gewicht der Feststoffe in der wässrigen Dispersion, ein oder mehrere Wirkstoffe, die Verbindungen mit biologischer Aktivität sind, ausgewählt aus der Gruppe, bestehend aus wasserlöslichen Wirkstoffen, partiell wasserlöslichen Wirkstoffen, wasserunlöslichen Wirkstoffen und Kombinationen davon.

2. Wässrige Dispersion gemäß Anspruch 1, wobei: (a) von 5 bis 49,9 Gew.-%, bezogen auf das Gewicht der Feststoffe in der wässrigen Dispersion, aus dem einen oder den mehreren Polymeren sind; (b) von 0,1 bis 45 Gew.-%, bezogen auf das Gewicht der Feststoffe in der wässrigen Dispersion, aus dem einen oder den mehreren Wirkstoffen sind; und ferner beinhaltend (c) zu von 94,9 bis 5,1 Gew.-%, bezogen auf das Gewicht der Feststoffe in der wässrigen Dispersion, ein oder mehrere Film bildende, durch die Polymerisation eines oder mehrerer ethylenisch ungesättigter Monomere hergestellte Polymere mit einem Zahlenmittel der Molmasse von mehr als 10 000; und wobei der eine oder die mehreren Wirkstoffe vor dem Kombinieren der Komponenten und Wasser in (a) verteilt werden.

3. Wässrige Dispersion gemäß Anspruch 1 oder Anspruch 2, wobei das eine oder die mehreren durch die Polymerisation eines oder mehrerer ethylenisch ungesättigter Monomere hergestellten Polymere mit einem Zahlenmittel der Molmasse von weniger als 10 000 ein Zahlenmittel der Molmasse von zwischen 1000 und 10 000 aufweisen.

4. Wässrige Dispersion gemäß einem der Ansprüche 1 bis 3, wobei das eine oder die mehreren durch die Polymerisation eines oder mehrerer ethylenisch ungesättigter Monomere hergestellten Polymere mit einem Zahlenmittel der Molmasse von weniger als 10 000 eine partielle oder keine Löslichkeit in Wasser aufweisen, so dass die aufgelöste Konzentration von Polymer in Wasser in dem Bereich von 25 bis 50 °C und bei einem pH von pH 2 bis pH 12 nicht mehr als 5 Gew.-% ist.

5. Eine Verwendung der wässrigen Dispersion gemäß Anspruch 2 bei der Herstellung eines Films oder eines Klebstoffs.

6. Ein Verfahren zum Freisetzen eines oder mehrerer Wirkstoffe aus einer wässrigen Dispersionszusammensetzung, beinhaltend: a) Herstellen einer ersten wässrigen Dispersion, die zu von 5 bis 49,9 Gew.-%, bezogen auf das Gewicht der Feststoffe in der wässrigen Dispersionszusammensetzung, ein oder mehrere durch die Polymerisation eines oder mehrerer ethylenisch ungesättigter Monomere hergestellte Polymere mit einem Zahlenmittel der Molmasse von weniger als 10 000 beinhaltet; b) Kombinieren der ersten wässrigen Dispersion mit 0,1 bis 45 Gew.-%, bezogen auf das Gewicht der Feststoffe in der wässrigen Dispersionszusammensetzung, von einem oder mehreren Wirkstoffen, die Verbindungen mit biologischer Aktivität sind, ausgewählt aus der Gruppe, bestehend aus wasserlöslichen Wirkstoffen, partiell wasserlöslichen Wirkstoffen, wasserunlöslichen Wirkstoffen und Kombinationen davon; und c) Kombinieren der ersten wässrigen Dispersion mit einer zweiten wässrigen Dispersion von 94,9 bis 5,1 Gew.-%, bezogen auf das Gewicht der wässrigen Dispersionszusammensetzung, von einem oder mehreren Film bildenden, durch die Polymerisation eines oder mehrerer ethylenisch ungesättigter Monomere hergestellten Polymeren mit einem Zahlenmittel der Molmasse von mehr als 10 000; wobei der eine oder die mehreren Wirkstoffe vor dem Kombinieren mit der zweiten wässrigen Dispersion in der ersten wässrigen Dispersion verteilt werden; und d) Trocknen der Mischung wässriger Dispersionen, um einen Film oder eine Beschichtung zu bilden.

7. Verfahren gemäß Anspruch 6, wobei nach dem Trocknen der Kombination der wässrigen Dispersionen ein Film gebildet wird.

## Revendications

1. Une dispersion aqueuse comprenant : a) de 5 à 99,9 % en poids, rapporté au poids de solides dans la dispersion aqueuse, d'un ou de plusieurs polymères, préparés par la polymérisation d'un ou de plusieurs monomères éthyléniquement insaturés, ayant une masse moléculaire moyenne en nombre inférieure à 10 000 ; et b) de 0,1 à 95 % en poids, rapporté au poids de solides dans la dispersion aqueuse, d'un ou de plusieurs ingrédients actifs qui sont des composés ayant une activité biologique sélectionnés dans le groupe constitué d'ingrédients actifs solubles dans l'eau, d'ingrédients actifs partiellement solubles dans l'eau, d'ingrédients actifs insolubles dans l'eau et de combinaisons de ceux-ci.

2. La dispersion aqueuse de la revendication 1 dans laquelle : (a) de 5 à 49,9 % en poids, rapporté au poids de solides dans la dispersion aqueuse, sont constitués de ce ou ces polymères ; (b) de 0,1 à 45 % en poids, rapporté au poids de solides dans la dispersion aqueuse, sont constitués de ce ou ces ingrédients actifs ; et comprenant en outre (c) de 94,9 à 5,1 % en poids, rapporté au poids de solides dans la dispersion aqueuse, d'un ou de plusieurs polymères filmogènes, préparés par la polymérisation d'un ou de plusieurs monomères éthyléniquement insaturés, ayant une masse moléculaire moyenne en nombre supérieure à 10 000 ; et dans laquelle ce ou ces ingrédients actifs sont séparés dans (a) préalablement à la combinaison des composants et de l'eau.

3. La dispersion aqueuse de la revendication 1 ou de la revendication 2 dans laquelle ce ou ces polymères, préparés par la polymérisation d'un ou de plusieurs monomères éthyléniquement insaturés, ayant une masse moléculaire moyenne en nombre inférieure à 10 000 ont une masse moléculaire moyenne en nombre comprise entre 1 000 et 10 000.

4. La dispersion aqueuse de n'importe lesquelles des revendications 1 à 3 dans laquelle ce ou ces dit(s) polymères, préparés par la polymérisation d'un ou de plusieurs monomères éthyléniquement insaturés, ayant une masse moléculaire moyenne en nombre inférieure à 10 000 ont une solubilité partielle ou aucune solubilité dans l'eau de telle sorte que la concentration dissoute de polymère dans l'eau dans la plage de 25 à 50 °C, et à un pH quelconque entre pH 2 et pH 12 ne soit pas supérieure à 5 % en poids.

5. Utilisation de la dispersion aqueuse de la revendication 2 dans la préparation d'un film ou d'un adhésif.

6. Une méthode pour libérer un ou plusieurs ingrédients actifs d'une composition de dispersion aqueuse comprenant : a) la préparation d'une première dispersion aqueuse comprenant de 5 à 49,9 % en poids, rapporté au poids de solides dans la composition de dispersion aqueuse, d'un ou de plusieurs polymères, préparés par la polymérisation d'un ou de plusieurs monomères éthyléniquement insaturés, ayant une masse moléculaire moyenne en nombre inférieure à 10 000 ; b) la combinaison de la première dispersion aqueuse avec de 0,1 à 45 % en poids, rapporté au poids de solides dans la composition de dispersion aqueuse, d'un ou de plusieurs ingrédients actifs qui sont des composés ayant une activité biologique sélectionnés dans le groupe constitué d'ingrédients actifs solubles dans l'eau, d'ingrédients actifs partiellement solubles dans l'eau, d'ingrédients actifs insolubles dans l'eau et de combinaisons de ceux-ci ; et c) la combinaison de la première dispersion aqueuse avec une deuxième dispersion aqueuse de 94,9 à 5,1 % en poids, rapporté au poids de la composition de dispersion aqueuse, d'un ou de plusieurs polymères filmogènes, préparés par la polymérisation d'un ou de plusieurs monomères éthyléniquement insaturés, ayant une masse moléculaire moyenne en nombre supérieure à 10 000 ; dans laquelle ce ou ces ingrédients actifs sont séparés dans la première dispersion aqueuse préalablement à la combinaison avec la deuxième dispersion aqueuse ; et d) le séchage du mélange de dispersions aqueuses afin de former un film ou un revêtement.

7. La méthode selon la revendication 6 dans laquelle un film est formé après séchage de la combinaison des dispersions aqueuses.
